**Europäisches Patentamt**

**European Patent Office**

**Office européen des brevets**

(11) Publication number : **0 481 725 A1**

# EUROPEAN PATENT APPLICATION

(21) Application number : **91309480.1**

(22) Date of filing : **15.10.91**

(51) Int. Cl.⁵ : **A61K 31/54,** A61K 47/00, A61K 47/10, A61K 47/14

(30) Priority : **19.10.90 JP 281095/90**

(43) Date of publication of application :
**22.04.92 Bulletin 92/17**

(84) Designated Contracting States :
**CH DE FR GB LI**

(72) Inventor : **Sawayanagi, Yoichi**
**c/o Dojin Iyaku-Kako Co.,Ltd., 2-2, Yayoi-cho**
**5-chome, Nakano-ku, Tokyo 164 (JP)**

(74) Representative : **Ben-Nathan, Laurence Albert**
**et al**
**Urquhart-Dykes & Lord 91 Wimpole Street**
**London W1M 8AH (GB)**

(71) Applicant : **DOJIN IYAKU-KAKO CO., LTD.**
**2-2, Yayoi-cho 5-chome**
**Nakano-ku, Tokyo 164 (JP)**

(54) **Piroxicam containing cream.**

㊗    An antiphlogistic analgesic cream composition comprising piroxicam of 0.1 ~5 weight %,
one or two or more kinds of emulsifiers of 10~25 weight % selected from polyoxyethylene alkyl ether, polyethylene glycol fatty acid ester and glycerol fatty acid ester,
a polyhydric alcohol of 4~12 weight %,
higher alcohol of 2~10 weight %, and
water of 48~83 weight %.
This antiphlogistic analgesic cream composition provides aging stability, less tackiness, satisfactory comfort in use and excellent antiphlogistic and analgesic effects.

EP 0 481 725 A1

## BACKGROUND OF THE INVENTION

The present invention relates to a composition of a cream preparation which contains piroxicam as an efficacious component and excels in antiphlogistic and analgesic actions with comfort in use and stability.

Piroxicam [chemical name: 4-hydroxy-2-methyl-N-(2-pyridyl)-2H-1, 2-benzothiazine-3-carboxamide-1, 1-dioxide] is widely used as an encapsulated drug or a suppository for articular rheumatism since it has an excellent antiphlogistic analgesic action.

However, it is generally known that the antiphlogistic analgesic preparation containing piroxicam as an efficacious component may induce a side effect such as a lesion of alimentary canals due to long term administration whereas it excels in efficacy. Accordingly, to relieve such side effect, it has been considered to use it as an external remedy to be externally applied.

It is known that piroxicam is transformed to a practically insoluble hydrate with presence of water to cause deposition and growth of crystal along with a lapse of time. As an external remedy using piroxicam, a gel ointment containing piroxicam prepared by dissolving it in alkanolamine and a cream preparation containing a monohydrate of piroxicam have been developed. However, the gel ointment has often been disliked since it generally contains a lot of lower alcohol to cause problems such as excessive skin stimulation and eruption, discomfort in use resulting from salting out and grimy exfoliation of the gelled preparation due to sweat after application, and special strange alcoholic fume.

On the other hand, piroxicam itself is insoluble in water but it is prone to be relatively dissolved in the oil phase of the cream preparation. Though it is assumed that piroxicam is emulsified after having been dissolved in the oil phase, there has been, in this case, a problem such as deposition and growth of crystal transformed to a hydrate after dissolving in the oil phase and therefore it has been inappropriate to prepare a cream preparation by dissolving piroxicam in the oil phase.

## SUMMARY OF THE INVENTION

For the above reason, the primary object of the present invention is to provide a cream preparation which contains stably compounded piroxicam and excels in antiphlogistic and analgesic actions with comfort in application to skins.

As a result of devoted studies to accomplish the above object, the inventor found that a cream preparation in which finely pulverized piroxicam (optimal average particle size is 10 μm or less) is dispersed with a special emulsifier and alcohol provides stable and excellent antiphlogistic analgesic action and a comfort in use without aging crystal growth of piroxicam and adoption of special manufacturing technology, and completed the present invention.

More specifically, the present invention provides an antiphlogistic analgesic cream composition composed of the following components shown in (a) to (e) below:

|  |  |
|---|---|
| (a) Piroxicam | $0.1 \sim 5$ weight % |
| (b) One or two or more kinds of emulsifiers selected from polyoxyethylene alkyl ether, polyethylene glycol fatty acid ester and glycerol fatty acid ester | $10 \sim 25$ weihgt % |
| (c) Polyhydric alcohol | $4 \sim 12$ weight % |
| (d) Higher alcohol | $2 \sim 10$ weight % |
| (e) Water | $48 \sim 83$ weight % |

## DESCRIPTION OF THE PREFERRED EMBODIMENTS

A compounding quantity of piroxicam which is component (a) used according to the present invention can

2

be determined to be within a range where its efficacy will be exhibited and shall be 0.1 ~ 5 weight %. If piroxicam which is an efficacious component is less than 0.1%, the antiphlogistic analgesic effect will be insufficient and, piroxicam exceeding 5% is disadvantageous in economical merits. Even in the above range, the range of 0.5~1.0 % is particularly preferable.

An emulsifier which is component (b) is composed with one or two or more kinds of emulsifiers selected from polyoxyethylene alkyl ether, polyethyleneglycol fatty acid ester and glycerol tatty acid ester. Polyoxyethylene alkyl ether preferably has polyoxyethylene group of 2~30 and alkyl group with the carbon number of 16~22 and, more specifically, is available as polyoxyethylene (20~25) cetyl ether, polyoxyethylene (10~30) behenyl ether, etc. Polyethyleneglycol fatty acid ester preferably has the number of ethylene oxide of 2 ~150 and the carbon number of principal fatty acid of 12~18 and, more specifically, is available as polyoxyethylene monostearate, polyoxyethylene distearate, polyoxyethylene monolaurate, polyoxyethylene dilaurate, etc. Glycerol fatty acid ester preferably has the carbon number of fatty acid of 14 ~18 and is available, for example, glyceryl monostearate, glyceryl monooleate, glyceryl myristate, etc. Though the cream preparation can be made with emulsifiers other than described above, it is clarified that it is slightly inferior in the edema inhibition ratio. The emulsifier of component (b) is preferably compounded as much as 10 ~25%, particularly 12 ~ 20%, in the whole preparation. If the quantity of this content is less than 10%, the efficacy cannot be fully exhibited and, if it exceeds 25%, the preparation will be discomfortable in use. Particularly for combination with this component (b), an emulsifier prepared with polyoxyethylene alkyl ether and polyethylene glycol fatty acid ester and/or glycerol fatty acid ester is preferable. In this case, a preferable compounding quantity of polyoxyethylene alkyl ether is 0.5 ~5%, particularly 1~3% in the whole preparation from the viewpoint of stability.

Polyhydric alcohol of component (c) is available as, for example, ethylene glycol, propylene glycol, butylene glycol, etc. The compounding quantity is 4 ~12%, preferably 5 ~8%. If the compounding quantity is less than 4% or exceeds 12%, the feeling in use will be worse and it is unfavorable.

Higher alcohol of component (d) is available as alcohol with the carbon number of 16 ~22 such as, for example, cetanol, stearyle alcohol, cetostearyle alcohol, etc. This higher alcohol of 2~10%, preferably 3 ~6%, is compounded to maitain physical stability of the cream preparation.

Water of component (e) of 48 ~83%, preferably 66~80%, is added.

The preparation in accordance with the present invention is usually prepared by, for example, heating oil phase components as required, emulsifying them with water phase components and adding piroxicam which is dispersed in polyhydric alcohol at 55~60° C. According to this preparation method, piroxicam can be used as free and need not be transformed to a monohydrate. Thus a stable cream preparation can be easily prepared.

Finely pulverized piroxicam of 10μm or less in average particle size is appropriate for actual use. The use of such pulverized particles is intended to improve adhesion to skins and percutaneous absorption.

When preparing the preparation according to the present invention, it is preferable for relieving excessive stimulation to skins to adjust the final pH value of the preparation to 4.0 ~ 7.0 by using a buffer such as a citric acid buffer solution which is commonly used in general external remedies.

Moreover, the preparation according to the present invention can be compounded as required with, in addition to the above components, aqueous component, powder. surface active agent, oil agent, humidity retaining agent, alcohols, antiseptics, coloring matters, antioxidant, ultraviolet rays absorbent, thickener, perfume, skin conditioner, etc. in appropriate quantities.

The antiphlogistic analgesic cream preparation according to the present invention as described above provides not only excellent medicinal effects but also aging stability with less tackiness and high extendibility.

## EXAMPLES

The following describes in more detail the present invention referring to the examples and test experiments; however the present invention is not limited to these examples.

In examples 1 to 3 and comparison examples 1 to 6, the antiphlogistic analgesic cream preparation or the antiphlogistic analgesic gel ointment is prepared in accordance with the compositions and methods shown respectively.

Example 1

Composition

| | |
|---|---|
| Piroxicam | 0.5 g |
| Cetostearyle alcohol | 3 g |
| Propylene glycol | 7 g |
| Polyoxyethylene cetyl ether (23 E.O.) | 2 g |
| Polyoxyethylene monostearate (40 E.O.) | 3 g |
| Polyoxyethylene monostearate (2 E.O.) | 4.8 g |
| Glyceryl monostearate | 3 g |
| Sodium citrate | 0.4 g |
| Citric acid | 0.28 g |
| Butyl P-hydroxybenzoate | 0.1 g |
| Methyl P-hydroxybenzoate | 0.2 g |

Purified water was added up to 100g as total quantity.

Preparing Method

The method of preparing the cream preparation was such that oil phase components were dissolved at 65 to 75 ° C, a water phase component in which other components were dissolved is added to it, this mixture was emulsified at 65 to 75 ° C, then piroxicam which was dispersed in propylene glycol was added at 55 to 60 ° C and thus the cream preparation was made by cooling it to room temperature while stirring it.

Example 2

Composition

| | |
|---|---|
| Piroxicam | 0.5 g |
| Stearyle alcohol | 4 g |
| Propylene glycol | 6 g |
| Polyoxyethylene cetyl ether (20 E.O.) | 2 g |
| Polyoxyethylene monostearate (40 E.O.) | 3.5 g |
| Polyoxyethylene monostearate (4 E.O.) | 14.5 g |

4

| Sodium citrate | 0.4 g |
| Citric acid | 0.28g |
| Butyl P-hydroxybenzoate | 0.1 g |
| Methyl P-hydroxybenzoate | 0.2 g |

Purified water was added up to 100g as total quantity.

Preparing Method

The cream preparation was similarly prepared as in Example 1.

Example 3

Composition

| Piroxicam | 0.5 g |
| Cetanol | 4 g |
| Propylene glycol | 7 g |
| Polyoxyethylene cetyl ether (23 E.O.) | 2 g |
| Polyethylene glycol fatty acid ester (8 E.O.) [Tradename: Kalaman] | 10 g |
| Sodium citrate | 0.4 g |
| Citric acid | 0.28g |
| Butyl P-hydroxybenzoate | 0.1 g |
| Methyl P-hydroxybenzoate | 0.2 g |

Purified water was added up to 100g as total quantity.

Preparing Method

The cream preparation was similarly prepared as in Example 1.

Comparison Example 1

Composition

| | |
|---|---|
| Piroxicam | 0.5 g |
| White vaseline | 15 g |
| Light liquid paraffin | 3 g |
| Stearic acid | 2.5 g |
| Bleached beeswax | 2 g |
| Sorbithane monostearate | 1.5 g |
| Polyoxyethylene sorbithane monostearate | 2 g |
| Cetyl sodium sulfate | 0.4 g |
| Citric acid | 0.28 g |
| Butyl P-hydroxybenzoate | 0.1 g |
| Methyl P-hydroxybenzoate | 0.2 g |

Purified water was added up to 100g as total quantity.

Preparing Method

Oil phase components were dissolved at 65 to 75° C, piroxicam was added to it, further a water phase component in which other components were dissolved was added to it, then this mixture was emulsified at 65 to 75° C and thus the cream preparation was made while cooling it to room temperature.

Comparison Example 2

Composition

| | |
|---|---|
| Piroxicam | 0.5 g |
| Cetostearyle alcohol | 3.5 g |
| Propylene glycol | 20 g |
| Polyoxyethylene cetyl ether (23 E.O.) | 2 g |
| Polyoxyethylene monostearate (40 E.O.) | 5 g |
| Polyoxyethylene monostearate (4 E.O.) | 25 g |
| Sodium citrate | 0.4 g |
| Citric acid | 0.28g |
| Butyl P-hydroxybenzoate | 0.1 g |
| Methyl P-hydroxybenzoate | 0.2 g |

Purified water was added up to 100g as total quantity.

Preparing Method

The cream preparation was similarly prepared as in Comparison Example 1.

Comparison Example 3

Composition

| | |
|---|---|
| Piroxicam | 0.5 g |
| Cetostearyle alcohol | 6.5 g |
| Polyoxyethylene cetyl ether (23 E.O.) | 2 g |
| Polyoxyethylene monostearate (40 E.O.) | 1 g |
| Polyoxyethylene monostearate (4 E.O.) | 5 g |
| Sodium citrate | 0.4 g |
| Citric acid | 0.28g |
| Butyl P-hydroxybenzoate | 0.1 g |
| Methyl P-hydroxybenzoate | 0.2 g |

Purified water was added up to 100g as total quantity.

Preparing Method

The cream preparation was similarly prepared as in Comparison Example 1.

Comparison Example 4

Composition

| | |
|---|---|
| Piroxicam | 0.5 g |
| White vaseline | 14 g |
| Solid paraffin | 2 g |
| Stearic acid | 5 g |
| Sorbithane monostearate | 3.5 g |
| Polyoxyethylene sorbithane monostearate | 3.5 g |
| Sodium citrate | 0.2 g |
| Citric acid | 0.14g |
| Butyl P-hydroxybenzoate | 0.1 g |
| Methyl P-hydroxybenzoate | 0.2 g |

Purified water was added up to 100g as total quantity.

Preparing Method

The cream preparation was similarly prepared as in Comparison Example 1.

Comparison Example 5

Composition

| | |
|---|---|
| Piroxicam | 0.5 g |
| White vaseline | 15 g |
| Bleached beeswax | 2 g |
| Stearic acid | 2.5 g |
| Light liquid paraffin | 3 g |
| Glyceryl monostearate | 1.5 g |
| Polyoxyethylene sorbithane monostearate | 2 g |
| Cetyl sodium sulfate | 0.4 g |
| Citric acid | 0.28g |
| Butyl P-hydroxybenzoate | 0.1 g |
| Methyl P-hydroxybenzoate | 0.2 g |

Purified water was added up to 100g as total quantity.

8

Preparing Method

The cream preparation was similarly prepared as in Comparison Example 1.

Comparison Example 6

Composition

| | |
|---|---|
| Piroxicam | 0.5 g |
| Caboxyvinyl polymer | 1.8 g |
| Diisopropanolamine | 2.9 g |
| Ethanol | 42.7 g |

Purified water was added up to 100g as total quantity.

Preparing Method

The above components were mixed and uniformly kneaded to prepare a gel ointment.

Experiment 1

Properties and separation stability at 50° C and 15 days later of the cream preparations obtained in the embodiments 1 to 3 and the comparison examples 1 to 3 were compared and examined. The results are shown in Table 1.

Table 1

| Sample | Property | Separation stability |
|---|---|---|
| Example 1 | Good cream preparation | ○ |
| Example 2 | Good cream preparation | ○ |
| Example 3 | Good cream preparation | ○ |
| Comparison example 1 | Good cream preparation | × |
| Comparison example 2 | Slightly soft cream preparation | ○ |
| Comparison example 3 | Lotionlike | × |

○ : Not separated    × : Separated

The cream preparations obtained in the examples 1 to 3 in accordance with the present invention excel in the properties and separation stability.

Experiment 2

With the cream preparations obtained in the examples 1 to 3 and a gel ointment obtained in the comparison example 6 as test samples, stimulation to skins was compared and examined on ten persons. A plaster for patch test to which the cream preparation or the gel ointment of 50mg was applied is adhered to an inside of a forearm, the plaster was removed 30 minutes later, the test sample was wiped off and the condition of the skin was observed immediately. The results are shown in Table 2.

### Table 2

| Sample | Determination (erythema occurrence ratio) |
|---|---|
| Example 1 | 0 / 10 |
| Example 2 | 1 / 10 |
| Example 3 | 0 / 10 |
| Comparison example 6 | 10 / 10 |

As known from Table 2, the cream preparation in accordance with the present invention showed a smaller stimulation to skin due to lower alcohol than the gel ointment.

Experiment 3

With cream preparations obtained in the examples 1 to 3 and the comparison examples 4 and 5 as test samples, antiphlogistic effects against edema of bruised paws of seven rats as a group were examined. Each cream preparation of 100mg was applied to surfaces of the hind paws and the paws were wrapped for two hours. A similar procedure was repeated two hours later, test samples were removed other two hours later and these foot soles were bruised. The edema inhibiting ratio 3 to 4 hours later was calculated. The results are as shown in Table 3.

### Table 3

| Sample | Edema inhibiting ratio (%) | |
|---|---|---|
| | 3 hours later | 4 hours later |
| Example 1 | 24.7 | 30.4 |
| Example 2 | 31.7 | 32.4 |
| Example 3 | 33.0 | 43.0 |
| Comparison example 4 | 10.1 | 9.8 |
| Comparison example 5 | 12.9 | 12.6 |
| Commercially-sold ointment | 31.5 | 36.3 |
| Non-treated (control) | – | – |

As known from Table 3, the cream preparation in accordance with the present invention which was applied to a group of rats showed a higher edema inhibiting ratio than other compared cream preparations and the same ratio equivalent to commercially sold piroxicam ointments.

Experiment 4

With the cream preparation according to the present invention obtained in the example 1 as a test sample, the clinical effectiveness and safety of the cream preparation according to the present invention were examined on the cases of diseases shown in Table 4 included in the range of orthopedic surgery. The results are as shown in Table 4. Resultant data as to side effects are as shown in Table 5.

Table 4   General Improvements

| | | R.I | I.I | S.I | U | S.W | I.W | R.W | T |
|---|---|---|---|---|---|---|---|---|---|
| Arthritis | N.C | 2 | 0 | 1 | 0 | 0 | 0 | 0 | 3 |
| deformans | C % | 66.7 | 66.7 | 100.0 | | | | | |
| Duplay's | N.C | 0 | 0 | 2 | 0 | 0 | 0 | 0 | 2 |
| disease | C.% | 0.0 | 0.0 | 100.0 | | | | | |
| Tendon/ten- | N.C | 2 | 2 | 0 | 1 | 0 | 0 | 0 | 5 |
| dosynovitis | C % | 40.0 | 80.0 | 80.0 | 100.0 | | | | |
| Peritendi- | N.C | 0 | 1 | 0 | 0 | 0 | 0 | 0 | 1 |
| nitis | C.% | 0.0 | 100.0 | | | | | | |
| Epicondy- | N.C | 0 | 1 | 0 | 0 | 0 | 0 | 0 | 1 |
| lalgia | C.% | 0.0 | 100.0 | | | | | | |
| Myosalgia | N.C | 2 | 1 | 0 | 0 | 0 | 0 | 0 | 3 |
| | C.% | 66.7 | 100.0 | | | | | | |
| Contusion/ | N.C | 3 | 1 | 0 | 0 | 0 | 0 | 0 | 4 |
| strain | C.% | 75.0 | 100.0 | | | | | | |
| Sprain | N.C | 1 | 0 | 0 | 0 | 0 | 0 | 0 | 1 |
| | C.% | 100.0 | | | | | | | |
| Fracture | N.C | 3 | 0 | 0 | 0 | 0 | 0 | 0 | 3 |
| | C.% | 100.0 | | | | | | | |
| Total | N.C | 13 | 6 | 3 | 1 | 0 | 0 | 0 | 23 |
| | C.% | 56.5 | 82.6 | 95.7 | 100.0 | | | | |

Note: Abbreviations in the above table denote as follows:

R.I: Remarkabe improvement   I.I: Intermediate improvement

S.I: Slight improvement   U: Unchanged   S.W: Slight worsening

I.W: Intermediate worsening   R.W: Remarkable worsening

T: Total

EP 0 481 725 A1

N.C: Number of cases     C %: Cumulative %

Table 5   Side Effect

|  | No | Yes | Total |
|---|---|---|---|
| Number of cases | 23 | 0 | 23 |
| Occurrence ratio % | 100.0 | 0.0 | 100.0 |

As known from Tables 4 and 5, the cream preparation in accordance with the present invention excels in efficacy and safety and the utility as a medicine was confirmed.

**Claims**

1.  An antiphlogistic analgesic cream composition comprising:

    piroxicam                                                          0.1 ～ 5 weight %

    one or two or more kinds of emulsifiers
    selected from polyoxyethylene alkyl ether,
    polyethylene glycol fatty acid ester and
    glycerol fatty acid ester                                          10 ～ 25 weihgt %

    polyhydric alcohol                                                 4 ～ 12 weight %

    higher alcohol                                                     2 ～ 10 weight %

    water                                                              48 ～ 83 weight %

2.  An antiphlogistic analgesic cream composition in accordance with Claim 1, wherein finely pulverized piroxicam of 10 μm or less in average particle size is used.

3.  An antiphlogistic analgesic cream composition comprising:

13

| | |
|---|---|
| piroxicam | 0.1 ~ 5 weight % |
| an emulsifier comprisng polyoxyethylene alkyl ether and polyethylene glycol fatty acid ester and/or glycerol fatty acid ester | 10 ~ 25 weihgt % |
| polyhydric alcohol | 4 ~ 12 weight % |
| higher alcohol | 2 ~ 10 weight % |
| water | 48 ~ 83 weight % |

4. An antiphlogistic analgesic cream composition in accordance with Claim 3, wherein finely pulverized piroxicam of 10 μm or less in average particle size is used.

5. An antiphlogistic analgesic cream composition in accordance with Claim 3, wherein a compounding quantity of said polyoxyethylene alkyl ether is within a range of 0.5 to 5 % of the whole quantity.

European Patent
Office

# EUROPEAN SEARCH REPORT

Application Number

EP 91 30 9480

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 179 430 (CHIESI FARMACEUTICI)<br>* Claims 1-4; page 7: "Cream formulation" *<br>--- | 1-5 | A 61 K 31/54<br>A 61 K 47/00<br>A 61 K 47/10<br>A 61 K 47/14 |
| Y | EP-A-0 313 347 (PROCTER & GAMBLE CO.)<br>* Claims 1-2,5-6; page 6, lines 62-65; page 7, lines 32-65; page 9, lines 1-16 *<br>--- | 1-5 | |
| Y | DATABASE WPIL, accession no. 86-289030 [44], Derwent Publications Ltd, London, GB; & JP-A-61 212 516 (TOYOBO K.K.) 20-09-1986<br>* Abstract *<br>----- | 1-5 | |

|  | TECHNICAL FIELDS SEARCHED (Int. Cl.5) |
|---|---|
|  | A 61 K |

The present search report has been drawn up for all claims

| Place of search | Date of completion of the search | Examiner |
|---|---|---|
| THE HAGUE | 26-11-1991 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

15